# FASCICULE DE BREVET EUROPEEN

(11) **EP 0 930 058 B1**
(45) Date de publication et mention de la délivrance du brevet: **27.02.2002**
(21) Numéro de dépôt: 98403261.5
(22) Date de dépôt: 22.12.1998
(51) Int. Cl.: A61K 7/00, A61K 7/42, A61K 7/48

(54) **Utilisation d'un solvant polyfluoré volatil, en tant qu'agent anti-transfert dans des produits cosmétiques**
Verwendung von einem flüchtigem Polyfluor-Lösungsmittel als übertragungsbeständiges Mittel in kosmetischen Produkten
Use of a volatile polyfluorinated solvent as a transfer resistant agent in cosmetical products

(30) Priorité: 29.12.1997 FR 9716628; 30.06.1998 FR 9808337
(43) Date de publication de la demande: 21.07.1999
(73) Titulaire: L'OREAL, 75008 Paris (FR)
(72) Inventeur: Bara, Isabelle, 75013 Paris (FR)
(74) Mandataire: Larcher, Dominique

(56) Documents cités:
- EP-A- 0 558 423
- EP-A- 0 595 683
- FR-A- 2 593 392
- FR-A- 2 756 176
- BASE DE DONN ES "CHEMICAL ABSTRACTS" (SERVEUR: STN): Abr.117:137 461, Colombus, OH, USA; & JP 04 139 121 A (TOSHIBA SILICONE CO.) 13 MAI 1992 XP002077150
- BASE DE DONN ES "CHEMICAL ABSTRACTS" (SERVEUR: STN): Abr.110: 160 222, Colombus, OH, USA; & JP 63 002 916 A (KANEBO, Ltd) 7 JANVIER 1988 XP002077151
- BASE DE DONN ES "CHEMICAL ABSTRACTS" (SERVEUR: STN): Abr.107:161 389, Colombus, OH, USA; & JP 62 093 211 A (SHISEIDO CO., Ltd) 28 AVRIL 1987 XP002086747

## Description

La présente invention a pour objet l'utilisation d'une certaine classe de composés polyhalogénoorganiques volatils, dont l'atome d'halogène est le fluor, dans des compositions cosmétiques de maquillage ou de protection solaire en vue d'éviter les phénomènes dits de "transfert".

Par "transfert" on entend dans ce type de compositions cosmétiques, le déplacement d'une fraction de la composition par contact avec un autre support, qu'il soit de même nature ou de nature différente. Ainsi, lorsque celles-ci sont destinées au maquillage telles que des fards à paupières, des eye-liners, ou des mascaras, elles peuvent se transférer sur les mains par frottement ou par contact de celles-ci avec les yeux. Lorsque ces compositions sont des rouges à lèvres, celles-ci peuvent se transférer sur les dents, les mains ou encore sur les joues d'une autre personne.

Quelles que soient le type de ces compositions, elles peuvent également par transfert, venir tâcher les serviettes de table et laisser des empreintes sur les verres, tasses et autres récipients.

En outre, lorsque les compositions sont des fonds de teint, un transfert peut également se produire du fond de teint sur le col des chemisiers et ainsi les tâcher.

En plus de ce problème de transfert, les compositions de maquillage peuvent également présenter des phénomènes de migration, c'est-à-dire que les compositions ont tendance à se propager à l'intérieur des ridules et/ou des rides de la peau, notamment dans le cas des fonds de teint, dans les ridules autour des lèvres dans le cas des rouges à lèvres et, dans les plis des paupières dans le cas des fards à paupières.

Ces phénomènes de transfert et de migration sont particulièrement préjudiciables dans la mesure où ils engendrent un effet inesthétique et un certain nombre d'inconvénients tels que mentionnés ci-dessus.

En vue d'y remédier, il a déjà été proposé., notamment dans le brevet US 5,505,937 d'utiliser des huiles volatiles notamment des silicones cycliques ainsi que des produits hydrocarbonés tels que des isoparaffines en C₈-C₂₀ associés à une résine de silicone et à des cires.

Si ce type de composition de maquillage permet d'éviter les phénomènes de transfert, il en résulte néanmoins la formation sur la peau d'un film sec, constitué de cires et de pigments d'aspect mat, lié à un état de surface du film hétérogène.

Il a également été proposé de réaliser des produits de maquillage sans transfert, en utilisant des dispersions aqueuses de polymères hydrophobes ou "latex" qui, en séchant sur la peau, permettent d'obtenir un film sec pouvant avoir une certaine brillance.

Néanmoins, l'inconvénient de ces dispersions aqueuses de polymères hydrophobes est lié à un inconfort et à une difficulté d'application sur la peau, notamment sur les lèvres dans le cas des rouges à lèvres.

Dans la demande EP 558 423, il a été décrit des dispersions huile-dans-l'eau susceptibles de former des films composites sur divers substrats kératiniques comprenant une phase fluorée constituée d'au moins un composé organofluoré hydrocarboné. Toutefois dans cette demande, le composé organofluoré hydrocarboné n'est pas du type volatil dans la mesure où il est présent dans le film composite conduisant à un toucher plus doux et plus agréable du film.

La demande de brevet EP-A-1 029 527 décrit l' utilisation cosmétique de solvants fluorés corresponda à la formule (II) selon l'invention. Cependant, leur utilisation en tant qu' agent anti-transfert n' est pas divulguée.

Après de nombreuses études sur divers types de composés, on a constaté de façon surprenante et inattendue, qu'en utilisant une certaine classe de solvants polyhalogénoorganiques volatils dont l'atome d'halogène est le fluor, il était possible de remédier, d'une façon particulièrement satisfaisante et efficace, aux inconvénients soulevés par les problèmes de transfert et de migration.

Un autre avantage particulièrement appréciable est de pouvoir obtenir les compositions en parfaite sécurité, dans la mesure où ces solvants, ci-après désignés par "solvants polyfluorés volatils", n'ont pas de point éclair, ce qui permet de réaliser les compositions à température élevée, et présente donc un avantage certain lorsque l'on utilise des substances ayant un point de fusion supérieur à la température ambiante.

Enfin, l'utilisation de solvants polyfluorés volatils facilite l'incorporation dans les compositions de concentrations élevées en dérivés fluorés non-volatils, ceux-ci étant totalement miscibles avec les solvants polyfluorés volatils. On peut ainsi obtenir un film résiduel hautement fluoré, brillant ou satiné, et résistant à l'eau et aux corps gras.

La présente invention a donc pour objet, l'utilisation dans une composition cosmétique de maquillage ou de protection solaire, contenant des particules de pigment et/ou de charge, d'au moins un solvant volatil, en tant qu'agent anti-transfert, et ledit solvant ayant une pression de vapeur supérieure à 20 mba (2000 Pa) à 25°C et de préférence supérieure à 40 mba (4000 Pa) et étant choisi parmi :
1°) le perfluorodiméthylcyclobutane et les composés perfluorocycloalkyles répondant à la formule (I) suivante : 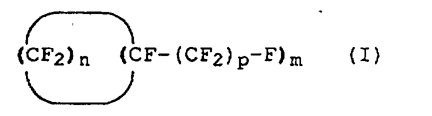 dans laquelle :
   n est 3, 4 ou 5,
   m est 1 ou 2, et
   p est 1, 2 ou 3
   sous réserve que lorsque m = 2, les groupements ne sont pas nécessairement en alpha, l'un par rapport à l'autre,
2°) les composés fluoroalkyles ou hétérofluoroalkyles répondant à la formule (II) suivante :

   CH₃-(CH₂)ₙ-[Z]ₜ-X-CF₃ (II)

   dans laquelle :
   t est 0 ou 1,
   n est 0, 1, 2 ou 3,
   X est un radical perfluoroalkyle divalent, linéaire ou ramifié, ayant de 2 à 5 atomes de carbone, et
   Z représente O, S, ou NR, R étant hydrogène, un radical -(CH₂)ₙ-CH₃ ou -(CF₂)ₘ-CF₃, m étant 2, 3, 4 ou 5,
3°) les dérivés de perfluomorpholine répondant à la formule (III) suivante : 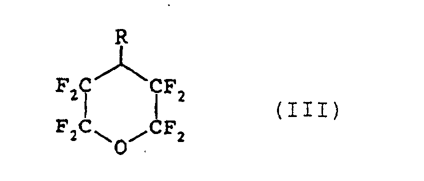 dans laquelle :
   R représente un radical perfluoroalkyle en C₁-C₄.

Parmi les composés perfluorocycloalkyles de formule (I), on peut notamment citer le perfluorométhylcyclopentane et le perfluorodiméthylcyclohexane, vendus respectivement sous les dénominations de "FLUTEC PC1® " et "FLUTEC PC3® " par la Société BNFL FLUOROCHEMICALS Ltd.

Parmi les composés fluoroalkyles ou hétérofluoroalkyles de formule (II) on peut notamment citer le méthoxynonafluorobutane vendu sous la dénomination de "HFE-7100®" par la Société 3M et l'éthoxynonafluorobutane vendu sous la dénomination de "HFE-7200® " par la Société 3M.

Parmi les dérivés de perfluoromorpholine de formule (III) on peut notamment citer la 4-trifluorométhyl perfluoromorpholine et la 4-pentafluoroéthyl perfluoromorpholine.

Les solvants polyfluorés volatils tels que définis ci-dessus doivent également satisfaire au critère du point d'ébullition, celui-ci devant être compris entre 20 et 75°C et de préférence entre 25 et 65°C.

Dans les compositions de maquillage ou de produit solaire, la proportion en solvant polyfluoré volatil est généralement comprise entre 2 et 98 % en poids, mais de préférence entre 5 et 70 % en poids par rapport au poids total de la composition.

Les pigments des compositions de maquillage ou de produit solaire ainsi que les charges, se présentent sous forme de particules très fines ayant une taille moyenne de particules comprise entre environ 0,02 à 50 µm.

Les pigments des compositions peuvent être minéraux ou organiques ou encore sous forme de laques métalliques. Parmi ces pigments on peut citer le dioxyde de titane, l'oxyde de zinc, le D&C Red No. 36 et le D&C Orange No. 17, les laques de calcium de D&C Red No. 7, 11, 31 et 34, la laque de baryum de D&C Red No. 12, la laque de strontium D&C Red No. 13, les laques d'aluminium de FD&C Yellow No. 5, de FD&C Yellow No. 6, de D&C Red No. 27, de D&C Red No. 21, de FD&C Blue No. 1, les oxydes de fer, le violet de manganèse, l'oxyde de chrome et le bleu d'outremer.

Les charges peuvent être d'origine naturelle ou synthétique. Parmi celles-ci, on peut notamment citer :
a) les poudres minérales telles que le talc, le kaolin, le mica, la silice, les silicates, l'alumine, les zéolites, l'hydroxyapatite, la séricite, les micatitanes, le sulfate de baryum, l'oxychlorure de bismuth, le nitrure de bore et les poudres métalliques telles que la poudre d'aluminium ;
b) les poudres végétales telles que les poudres d'amidon, de mais, de froment ou de riz ;
c) les poudres organiques telles que les poudres de nylon, de polyamide, de polyester, de polytétrafluoroéthylène ou de polyéthylène.

Ces différentes poudres peuvent en outre être enrobées, par exemple, par des sels métalliques d'acides gras, des acides aminés, de la lécithine, du collagène, des composés siliconés, des composés fluorés ou par tout autre enrobage usuel.

En plus des pigments tels que définis ci-dessus, les compositions peuvent en outre contenir, au moins un colorant et, parmi ceux-ci, on peut citer les dérivés d'éosine tel que le D&C Red No. 21 et les dérivés de fluoroscéine halogéné tel que le D&C Red No. 27, le D&C Orange No. 5 en association avec le D&C Red No. 21 et le D&C Orange No. 10.

Dans les compositions de maquillage ou de produit solaire, la proportion en au moins un pigment et/ou colorant est généralement comprise entre environ 0,1 et 15 % en poids par rapport au poids total de la composition.

Les charges peuvent être généralement présentes dans les produits de maquillage ou solaires en une proportion maximum d'environ 98 % en poids par rapport au poids total de la composition.

Selon un premier mode de réalisation particulier des compositions de maquillage ou de produit solaire, celles-ci sont anhydres et comprennent une phase grasse en une proportion comprise entre environ 0,3 à 90 % en poids par rapport au poids total de la composition.

La phase grasse est généralement constituée d'un ou plusieurs corps gras qui peuvent être choisis parmi les huiles, les cires, les gommes et/ou les corps gras dits pâteux.

**A** - Les huiles de la phase grasse peuvent être d'origine minérale, animale, végétale ou de synthèse, celles-ci pouvant être volatiles ou non à température ambiante.

Comme huile d'origine minérale, on peut citer notamment l'huile de paraffine et l'huile de vaseline.

Comme huile d'origine animale, on peut citer notamment le squalane ou perhydrosqualène.

Comme huile d'origine végétale, on peut citer notamment l'huile d'amande douce, l'huile de calophyllum, l'huile de palme, l'huile d'avocat, l'huile de jojoba, l'huile de sésame, l'huile d'olive, l'huile de ricin et les huiles de germes de céréales telles que par exemple l'huile de germes de blé.

Comme huile de synthèse, on peut citer notamment :
(1) les esters de formule suivante :

   R₁-COOR₂

   dans laquelle :
   R₁ représente le reste d'un acide gras supérieur ayant de 7 à 20 atomes de carbone et
   R₂ représente un radical hydrocarboné ayant de 3 à 30 atomes de carbone.
      Parmi ces esters, on peut notamment citer : l'huile de Purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, l'isononanoate d'isononyle, les esters dérivés de l'acide lanolique tels que le lanolate d'isopropyle et le lanolate d'isocétyle.
      Comme autres huiles de synthèse, on peut citer en outre l'isododécane, l'isohexadécane, les polyisobutènes et le polyisobutène hydrogéné ainsi que les acétylglycérides, les octanoates et décanoates de polyalcools tels que ceux de glycol et de glycérol, les ricinoléates d'alcools et de polyalcools tels que celui d'alcool cétylique, le dicaprylate de propylène glycol et l'adipate de diisopropyle ;
   (2) les alcools gras tels que l'alcool oléique, l'alcool linoléique, l'alcool linolénique, l'alcool isostéarylique et l'octyldodécanol ;
   (3) les huiles de silicone telles que les polydiorganosiloxanes linéaires éventuellement fonctionnalisés, les polydiorganosiloxanes cycliques et en particulier les cyclotétra- et penta-diméthicones et les organopolysiloxanes tels que les alkyl, alcoxy ou phényl diméthicones et en particulier la phényltriméthicone ;
   (4) les huiles fluorées non volatiles telles que la perfluorodécaline, le perfluorophénanthrène, les perfluoroalcanes et les perfluoropolyéthers et les huiles hydrocarbonées partiellement fluorées.

Selon une forme particulièrement préférée de l'invention, on utilise des perfluoropolyéthers non volatils répondant aux formules (IV) et (V) suivantes : 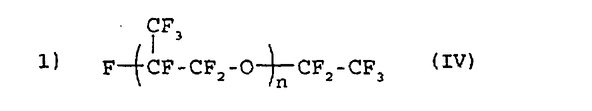 dans laquelle :
n = 7 à 30, 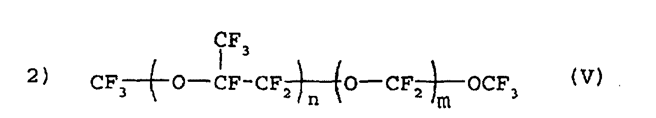 dans laquelle :
n et m = 20 à 40.

Parmi ceux-ci on peut citer ceux vendus sous les dénominations de "FOMBLIN C® " et de "GALDEN® " par la Société AUSSIMONT, ou encore sous la dénomination "FLUORTRESS LM 36® " par la Société DU PONT.

**B** - Les cires de la phase grasse peuvent être d'origine minérale, fossile, animale, végétale, de synthèse ou bien encore être des huiles hydrogénées ou des esters gras concrets à 25°C.

Parmi les cires minérales, on peut citer notamment les cires microcristallines, la paraffine, la vaseline et la cérésine.

Parmi les cires fossiles, on peut citer l'ozokérite et la cire de montan.

Parmi les cires d'origine animale, on peut citer la cire d'abeilles, le spermaceti, la cire de lanoline ainsi que les dérivés issus de la lanoline tels que les alcools de lanoline, la lanoline hydrogénée, la lanoline hydroxylée, la lanoline acétylée, les acides gras de lanoline et l'alcool de lanoline acétylé.

Parmi les cires d'origine végétale, on peut citer notamment la cire de candellila, la cire de carnauba, la cire du Japon et le beurre de cacao.

Parmi les cires de synthèse, on peut citer notamment les homopolymères d'éthylène et les copolymères d'éthylène et d'un monomère répondant à la formule (VI) suivante :

CH₂=CH-R₃ (VI)

dans laquelle :
R₃ représente un radical alkyle ayant de 1 à 30 atomes de carbone, un radical aryle ou aralkyle.

Le radical alkyle ayant de 1 à 30 atomes de carbone est de préférence le radical méthyle, éthyle, propyle, isopropyle, butyle, décyle, dodécyle ou octadécyle.

On peut également utiliser les cires obtenues par synthèse Fisher-Tropsch ainsi que les cires de silicone.

Parmi les huiles hydrogénées concrètes à 25°C, on peut citer notamment l'huile de ricin hydrogénée, l'huile de palme hydrogénée, le suif hydrogéné et l'huile de coco hydrogénée.

Parmi les esters gras concrets à 25°C, on peut citer notamment le mono-myristate de propylène glycol et le myristate de myristyle.

Comme cires utilisables dans les compositions selon l'invention, on peut encore citer l'alcool cétylique, l'alcool stéarylique, les mono-, di- et triglycérides concrets à 25°C, le monoéthanolamide stéarique, la colophane et ses dérivés tels que les abiétates de glycol et de glycérol, les sucro-glycérides et les oléates, myristates, lanolates, stéarates et dihydroxystéarates de calcium, de magnésium, de zinc, d'aluminium et les cires fluorées.

C - Les corps gras de type pâteux peuvent être d'origine minérale, animale, végétale ou de synthèse.

Parmi les corps gras pâteux, on peut citer notamment les esters de synthèse tels que le propionate d'arachidyle, le polylaurate de vinyle, les cires de polyéthylène et les organopolysiloxanes tels que les alkyldiméthicones, les alcoxydiméthicones ou les esters diméthicones.

Les compositions anhydres telles que définies ci-dessus, peuvent bien entendu contenir en outre un ou plusieurs additifs ou adjuvants cosmétiques ou dermatologiques conventionnels.

Ces compositions anhydres peuvent se présenter sous différentes formes telles que, par exemple, d'un gel huileux, d'un produit solide tel qu'une poudre compactée ou coulée, ou bien encore d'un stick tel que par exemple d'un rouge à lèvres.

Lorsque les compositions sont sous forme d'un gel huileux, elles contiennent généralement, outre les constituants définis précédemment, un gélifiant huileux.

Parmi les gélifiants huileux, on peut notamment citer les esters métalliques tels que le stéarate de polyoxyaluminium et l'hydroxystéarate d'aluminium ou de magnésium, les esters d'acide gras et de glycol, les triglycérides, les mélanges d'alcools gras, les dérivés de cholestérol et en particulier l'hydroxycholestérol, et les minéraux argileux gonflants en présence d'huile et en particulier ceux appartenant au groupe des montmorillonites.

Les gélifiants huileux peuvent être présents en une proportion très variable selon la texture des compositions recherchée. Toutefois, dans la plupart des cas, ils sont présents en une proportion comprise entre environ 0,1 et 30 % en poids par rapport au poids total de la composition.

Ces compositions anhydres peuvent être en particulier des fonds de teint, des mascaras, des eye-liners, des rouges à lèvres, des fards à paupières ou à joues.

Selon un deuxième mode de réalisation des compositions selon l'invention, celles-ci sont des dispersions, sous forme d'une émulsion stable eau-dans-l'huile (E/H) ou huile-dans-l'eau (H/E) qui sont essentiellement constituées (i) d'une phase grasse en une proportion comprise entre environ 0,1 et 50 % en poids par rapport au poids total de la composition, ladite phase grasse pouvant contenir au moins un corps gras tel que défini ci-dessus en une proportion comprise entre environ 0,1 et 95 % en poids par rapport au poids total de la composition, (ii) d'une phase aqueuse en une proportion comprise entre environ 4 et 97 % en poids par rapport au poids total de la composition, et (iii) d'au moins un agent émulsionnant en une proportion comprise entre environ 1 et 10 % en poids par rapport au poids total de la composition sous forme d'émulsion.

Comme agent émulsionnant ou tensioactif pouvant être utilisé dans les compositions sous forme d'une émulsion E/H ou H/E, on peut citer notamment les agents tensioactifs siliconés, et en particulier ceux appartenant à la famille des alkyl- ou alcoxydiméthicone copolyols. Parmi les alkyl- ou alcoxydiméthicone copolyols, on peut citer notamment les composés répondant à la formule générale suivante : 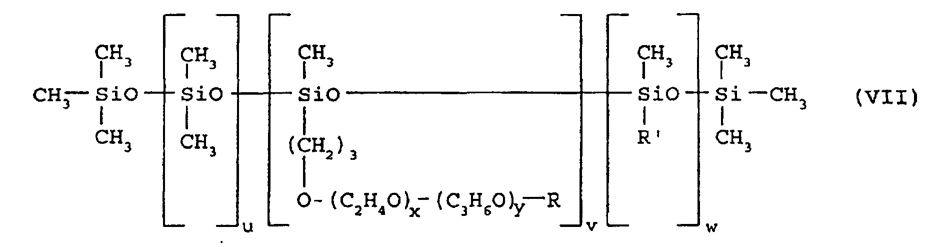 dans laquelle :
R est un atome d'hydrogène, un alkyle en C₁-C₁₆, un alcoxy ou acyle,
R' est un radical alkyle ou alcoxy en C₈-C₂₂,
u = 0 à 200,
v = 1 à 40,
w = 1 à 100,
le poids moléculaire du radical -O- (C₂H₄O)ₓ― (C₃H₆O)_{y}―R étant de 250 à 2000, x et y étant choisis de telle sorte que le rapport en poids des groupes oxyéthylène/oxypropylène soit compris entre 100:0 et 20:80.

Parmi les produits du commerce pouvant contenir tout ou partie des alkyldiméthicones copolyols, on peut citer notamment ceux vendus sous les dénominations de "ABIL WE09® ", "ABIL EM90® " ou "ABIL WS08® " par la Société GOLDSCHMIDT, de "Q2 5200® " ou "Q2 3225C® " par la Société DOW CORNING et de "218 1138® " par la Société GENERAL ELECTRIC.

Les agents tensioactifs peuvent être également choisis parmi des agents tensioactifs anioniques ou non ioniques. On peut à ce sujet se reporter au document "Encyclopedia of Chemical Technology, KIRK-OTHMER", volume 22, pages 333-432, 3^{ème} édition, 1979 WILEY, pour la définition des propriétés et des fonctions (émulsionnant) des tensioactifs, en particulier pages 347-377 de cette référence, pour les tensioactifs anioniques et non ioniques.

Les tensioactifs de ces deux groupes utilisés de préférence dans les compositions selon l'invention sont :
- parmi les tensioactifs non ioniques : les acides gras, les alcools gras, les alcools gras polyéthoxylés ou polyglycérolés, tels que les alcools stéarylique ou cétylstéarylique polyéthoxylés, les esters d'acides gras et de saccharose, les esters alkyl glucose, en particulier les esters gras d'alkyl(C₁-C₆)glucose polyoxyéthylénés, et
- parmi les tensioactifs anioniques : les stéarates d'amines.

Ces émulsions se présentent de préférence sous forme de crèmes et peuvent être utilisées comme produits de maquillage ou produits solaires. Dans ce dernier cas, elles contiennent des filtres solaires UVA et/ou UVB ainsi que des pigments blancs, en une proportion variable selon le degré de protection recherché.

Les compositions telles que décrites ci-dessus, qu'elles soient du type anhydre ou sous forme d'une dispersion, présentent d'excellentes propriétés cosmétiques telles que notamment une excellente facilité d'application, une grande douceur et conduisent à l'obtention d'un maquillage homogène.

Les compositions telles qu'elles viennent d'être décrites ci-dessus, peuvent en outre contenir un ou plusieurs adjuvants cosmétiques conventionnels tels que des vitamines, des hormones, des agents antioxydants, des conservateurs, des parfums, des épaississants, des agents hydratants, des agents humectants, des polymères anioniques, non-ioniques ou amphotères, ou des actifs cosmétiques ou dermatologiques.

L'invention sera maintenant illustrée par les différents exemples suivants, dont les quantités sont exprimées en poids.

### EXEMPLES

### EXEMPLE 1 : Rouge à lèvres résistant au transfert

- Méthoxynonafluorobutane (HFE-7100®) 10 g
- Perfluorodécaline. 35 g
- Cire 40 g
- Polyperfluoroisopropyléther (FLUORTRESS LM 36® ) 5 g
- Pigments 10 g

Après mélange à chaud des corps gras, à une température inférieure à celle d'évaporation de l'huile fluorée volatile, on introduit sous agitation l'huile volatile et les pigments, et on verse ensuite le mélange fondu dans des moules à rouges à lèvres.

Après refroidissement et démoulage, on obtient des rouges à lèvres de bonne texture ayant une bonne facilité d'application et un temps de séchage très court. On constate par ailleurs, qu'après séchage, le rouge à lèvres ne donne lieu à aucun phénomène de transfert selon les tests conventionnels utilisés pour ce type de détermination.

Dans cet exemple, le méthoxynonafluorobutane peut être avantageusement remplacé par une quantité équivalente de perfluorodiméthylcyclohexane.

### EXEMPLE 2 : Fond de teint

- Perfluorométhylcyclopentane (FLUTEC PC 1®) 20 g
- Alkyldiméthicone copolyol (ABIL WE 09®) 5 g
- Cyclométhicone 10 g
- Pigments 7 g
- Eau q.s.p. 100 g

Ce fond de teint est obtenu sous forme d'une émulsion eau-dans-l'huile (E/H) par mélange sous agitation de la phase grasse et de la phase aqueuse. On obtient ainsi un fond de teint de bonne consistance, qui s'applique facilement. Il est particulièrement résistant au frottement.

Dans cet exemple, le perfluorométhylcyclopentane peut être avantageusement remplacé par une quantité équivalente d'éthoxynonafluorobutane ou de 4-trifluorométhyl octafluoromorpholine.

## Revendications

1. Utilisation dans une composition cosmétique de maquillage ou de protection solaire, contenant des particules de pigment et/ou de charge, d'au moins un solvant volatil, en tant qu'agent anti-transfert, ledit solvant ayant une pression de vapeur supérieure à 20 mba (2000 Pa) à 25°C et étant choisi parmi :
(i) le perfluorodiméthylcyclobutane ou un composé perfluorocycloalkyle répondant à la formule suivante : 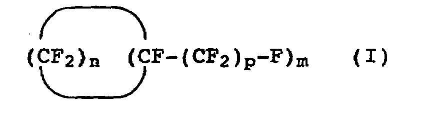 dans laquelle :
n est 3, 4 ou 5,
m est 1 ou 2, et
p est 1, 2 ou 3
sous réserve que lorsque m = 2, les groupements ne sont pas nécessairement en alpha, l'un par rapport à l'autre,
(ii) un composé fluoroalkyle ou hétérofluoroalkyle répondant à la formule suivante :
CH₃-(CH₂)ₙ-[Z]ₜ-X-CF₃ (II)
dans laquelle :
t est 0 ou 1,
n est 0, 1, 2 ou 3,
X est un radical perfluoroalkyle divalent, linéaire ou ramifié, ayant de 2 à 5 atomes de carbone, et
Z représente O, S, ou NR, R étant hydrogène, un radical -(CH₂)ₙ-CH₃ ou -(CF₂)ₘ-CF₃, m étant 2, 3, 4 ou 5, et
(iii) un dérivé de perfluoromorpholine répondant à la formule : 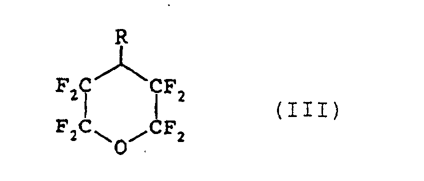 dans laquelle :
R représente un radical perfluoroalkyle en C₁-C₄.

2. Utilisation selon la revendication 1, **caractérisée par le fait que** le composé perfluorocycloalkyle est choisi dans le groupe constitué par le perfluorométhylcyclopentane et le perfluorodiméthylcyclohexane.

3. Utilisation selon la revendication 1 **caractérisée par le fait que** le composé hétérofluoroalkyle répond à la formule : CH₃ - (CH₂)ₙ -[Z]ₜ-X -CF₃ dans laquelle :
t = 1
n est 0, 1, 2 ou 3
X est un radical perfluoroalkyle divalent, linéaire ou ramifié, ayant de 2 à 5 atomes de carbone.
et Z représente O.

4. Utilisation selon la revendication 1 ou 3, **caractérisée par le fait que** le composé hétérofluoroalkyle est le méthoxynonafluorobutane ou l'éthoxynonafluorobutane.

5. Utilisation selon la revendication 1, **caractérisée par le fait que** le dérivé de perfluoromorpholine est la 4-trifluorométhyl perfluoromorpholine et la 4-pentafluoroéthyl perfluoromorpholine.

6. Utilisation selon l'une quelconque des revendications précédentes, **caractérisée par le fait que** le solvant volatil est présent dans la composition en une proportion comprise entre 2 et 98 % en poids par rapport au poids total de la composition.

7. Utilisation selon l'une quelconque des revendications précédentes, **caractérisée par le fait que** la composition contient en outre au moins un colorant.

8. Utilisation selon l'une quelconque des revendications précédentes, **caractérisée par le fait que** la proportion en pigment dans la composition, en présence éventuelle d'un colorant, est comprise entre 0,1 et 15 % en poids par rapport au poids total de la composition.

9. Utilisation selon l'une quelconque des revendications précédentes, **caractérisée par le fait que** la proportion en charge dans la composition est au maximum de 98 % en poids par rapport au poids total de la composition.

10. Utilisation selon l'une quelconque des revendications précédentes, **caractérisée par le fait que** la composition cosmétique est anhydre et se présente sous forme d'un fond de teint, d'un mascara, d'un eye-liner, d'un rouge à lèvres, d'un fard à paupières ou à joues pour le maquillage.

11. Utilisation selon l'une quelconque des revendications 1 à 10, **caractérisée par le fait que** la composition cosmétique est une émulsion eau-dans-l'huile ou huile-dans-l'eau et se présente sous forme d'une crème pour le maquillage ou pour la protection solaire.

## Patentansprüche

1. Verwendung mindestens eines flüchtigen Lösungsmittels als Antiübertragungsmittel in einer kosmetischen Schmink- oder Sonnenschutzzubereitung, enthaltend Pigment- und/ oder Füllstoffteilchen, wobei das Lösungsmittel einen Dampfdruck oberhalb von 20 mbar (2000 Pa) bei 25 °C aufweist und ausgewählt wird unter:
(i) Perfluordimethylcyclobutan oder einer Perfluorcycloalkylverbindung, die der folgenden Formel entspricht: 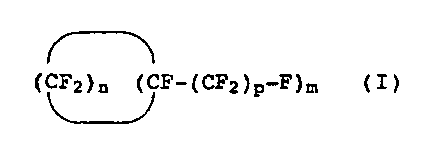 wobei:
n 3, 4 oder 5 ist,
m 1 oder 2 ist, und
p 1, 2 oder 3 ist,
unter dem Vorbehalt, dass bei m = 2 die Gruppen, die eine im Vergleich zur anderen, nicht notwendigerweise in Alpha-Position zueinander vorliegen,
(ii) einer Fluoralkyl- oder Heterofluoralkylverbindung, die der folgenden Formel entspricht:
CH₃-(CH₂)ₙ-[Z]ₜ-X-CF₃ (II)
wobei:
t 0 oder 1 ist,
n 0, 1, 2 oder 3 ist,
X ein bivalenter, linearer oder verzweigter Perfluoralkylrest mit 2 bis 5 Kohlenstoffatomen ist, und
Z O, S oder NR darstellt, wobei R Wasserstoff, -(CH₂)ₙ-CH₃- oder -(CF₂)ₘCF₃- ist und wobei m 2, 3, 4 oder 5 ist, und
(iii) einem Perfluormorpholinderivat, das der folgenden Formel entspricht: 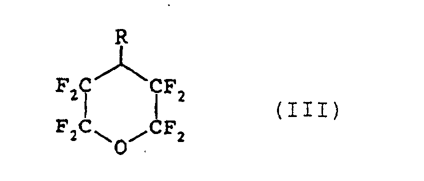 wobei:
R ein C₁-C₄-Perfluoralkylrest ist.

2. Verwendung nach Anspruch 1, **dadurch gekennzeichnet, dass** die Perfluorcycloalkylverbindung ausgewählt wird aus der Gruppe bestehend aus Perfluormethylcyclopentan und Perfluordimethylcyclohexan.

3. Verwendung nach Anspruch 1, **dadurch gekennzeichnet, dass** die Heterofluoralkylverbindung der folgenden Formel entspricht:
CH₃ - (CH₂)ₙ - [Z]ₜ-X -CF₃
wobei:
t = 1,
n 0, 1, 2 oder 3 ist,
X ein bivalenter, linearer oder verzweigter Perfluoralkylrest mit 2 bis 5 Kohlenstoffatomen ist, und
Z O darstellt.

4. Verwendung nach Anspruch 1 oder 3, **dadurch gekennzeichnet, dass** die Heterofluoralkylverbindung Methoxynonafluorbutan oder Ethoxynonafluorbutan ist.

5. Verwendung nach Anspruch 1, **dadurch gekennzeichnet, dass** das Perfluormorpholinderivat 4-Trifluormethylperfluormorpholin und 4-Pentafluorethylperfluormorpholin ist.

6. Verwendung nach einem der vorstehenden Ansprüche, **dadurch gekennzeichnet, dass** das flüchtige Lösungsmittel in der Zubereitung in einem Anteil von 2 bis 98 Gew.-% vorliegt, bezogen auf das Gesamtgewicht der Zubereitung.

7. Verwendung nach einem der vorstehenden Ansprüche, **dadurch gekennzeichnet, dass** die Zubereitung außerdem mindestens einen Farbstoff enthält.

8. Verwendung nach einem der vorstehenden Ansprüche, **dadurch gekennzeichnet, dass** der Pigmentanteil in der Zubereitung, gegebenenfalls in der Anwesenheit eines Farbstoffs, 0,1 bis 15 Gew.-% beträgt, bezogen auf das Gesamtgewicht der Zubereitung.

9. Verwendung nach einem der vorstehenden Ansprüche, **dadurch gekennzeichnet, dass** der Anteil an Füllstoff in der Zubereitung maximal 98 Gew.-% beträgt, bezogen auf das Gesamtgewicht der Zubereitung.

10. Verwendung nach einem der vorstehenden Ansprüche, **dadurch gekennzeichnet, dass** die kosmetische Zubereitung wasserfrei ist und in Form von Makeup bzw. einer Grundierung, Wimperntusche, einem Eyeliner, Lippenstift, Lidschatten oder Rouge vorliegt.

11. Verwendung nach einem der Ansprüche 1 bis 10, **dadurch gekennzeichnet, dass** die kosmetische Zubereitung eine Wasser-in-Öl- oder Öl-in-Wasser-Emulsion darstellt und in Form einer Creme für das Schminken bzw. Makeup oder für den Sonnenschutz vorliegt.

## Claims

1. Use, in a cosmetic make-up or antisun composition containing pigment and/or filler particles, of at least one volatile solvent as transfer-resistance agent, the said solvent having a vapour pressure of greater than. 20 mba (2 000 Pa) at 25°C and being chosen from:
(i) perfluorodimethylcyclobutane or a perfluorocycloalkyl compound corresponding to the following formula: 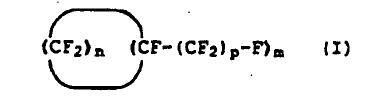 in which:
n is 3, 4 or 5,
m is 1 or 2, and
p is 1, 2 or 3
with the proviso that when m = 2, the groups are not necessarily alpha to each other,
(ii) a fluoroalkyl or heterofluoroalkyl compound corresponding to the following formula:
CH₃-(CH₂)ₙ-[Z]ₜ-X-CF₃ (II)
in which:
t is 0 or 1,
n is 0, 1, 2 or 3,
X is a linear or branched divalent perfluoroalkyl radical containing from 2 to 5 carbon atoms, and
Z represents O, S or NR, R being hydrogen, a radical -(CH₂)ₙ-CH₃ or -(CF₂)ₘ-CF₃, m being 2, 3, 4 or 5, and
(iii) a perfluoromorpholine derivative corresponding to the formula: 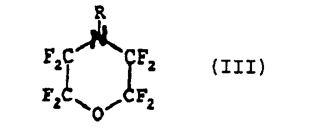 in which:
R represents a C₁-C₄ perfluoroalkyl radical.

2. Use according to Claim 1, **characterized in that** the perfluorocycloalkyl compound is chosen from the group consisting of perfluoromethylcyclopentane and perfluorodimethylcyclohexane.

3. Use according to Claim 1, **characterized in that** the heterofluoroalkyl compound corresponds to the formula:
CH₃-(CH₂)ₙ-[Z]ₜ-X-CF₃
in which:
t = 1
n is 0, 1, 2 or 3
X is a linear or branched divalent perfluoroalkyl radical containing from 2 to 5 carbon atoms, and
Z represents O.

4. Use according to Claim 1 or 3, **characterized in that** the heterofluoroalkyl compound is methoxynonafluorobutane or ethoxynonafluorobutane.

5. Use according to Claim 1, **characterized in that** the perfluoromorpholine derivative is 4-trifluoromethylperfluoromorpholine or 4-pentafluoroethylperfluoromorpholine.

6. Use according to any one of the preceding claims, **characterized in that** the volatile solvent is present in the composition in a proportion of between 2% and 98% by weight relative to the total weight of the composition.

7. Use according to any one of the preceding claims, **characterized in that** the composition also contains at least one colorant.

8. Use according to any one of the preceding claims, **characterized in that** the proportion of pigment in the composition, in the optional presence of a colorant, is between 0.1% and 15% by weight relative to the total weight of the composition.

9. Use according to any one of the preceding claims, **characterized in that** the proportion of filler in the composition is not more than 98% by weight relative to the total weight of the composition.

10. Use according to any one of the preceding claims, **characterized in that** the cosmetic composition is anhydrous and is in the form of a make-up foundation, mascara, eyeliner, lipstick, eyeshadow or face powder.

11. Use according to any one of Claims 1 to 10, **characterized in that** the cosmetic composition is a water-in-oil or oil-in-water emulsion and is in the form of a make-up cream or antisun cream.
